# EUROPÄISCHE PATENTSCHRIFT

(11) **EP 0 710 650 B1**
(45) Veröffentlichungstag und Bekanntmachung des Hinweises auf die Patenterteilung: **26.08.1998**
(21) Anmeldenummer: 95116937.4
(22) Anmeldetag: 27.10.1995
(51) Int. Cl.: C07D 213/89

(54) **Verfahren zur Herstellung von 1-Hydroxy-2-pyridonen**
Process for the preparation of 1-hydroxy-2-pyridones
Procédé pour la préparation de 1-hydroxy-2-pyridones

(30) Priorität: 02.11.1994 DE 4439029; 16.05.1995 DE 19517891
(43) Veröffentlichungstag der Anmeldung: 08.05.1996
(73) Patentinhaber: HOECHST AKTIENGESELLSCHAFT, 65929 Frankfurt am Main (DE)
(72) Erfinder: Peyman, Anuschirwan, Dr., D-65779 Kelkheim (DE); Reuschling, Dieter Bernd, Dr., D-35510 Butzbach (DE); Linkies, Adolf Heinz, Dr., D-65929 Frankfurt (DE)

(56) Entgegenhaltungen:
- DE-C- 3 626 210
- FR-A- 2 177 963

## Beschreibung

Nach dem in DE 2 214 608 beschriebenen Verfahren werden 1-Hydroxy-2-pyridone durch Umsetzung der entsprechenden 2-Pyrone (Formel II) mit Hydroxylamin oder einem seiner Salze in Gegenwart eines - gegebenenfalls substituierten - Aminopyridins oder Imidazols hergestellt. Das Aminopyridin oder Imidazol wird hierbei vorteilhaft in wenigstens äquimolarer Menge, bezogen auf das Hydroxylammoniumsalz, eingesetzt. Als Temperaturbereich werden Temperaturen zwischen 50 °C und 120 °C angegeben. Bei der Umsetzung erfolgt ein Austausch des Ringsauerstoffatoms des 2-Pyrons durch die N-OH-Gruppe. Die Ausbeuten liegen zwischen 60 % und 70 % der Theorie, bezogen auf eingesetztes 2-Pyron. Dieses Verfahren hat den Nachteil, daß beträchtliche Mengen der relativ wertvollen und teuren Aminopyridine und/oder Imidazole verwendet werden, die wegen ihres beträchtlichen Wertes und auch aus Umweltschutzgründen wieder zurückgewonnen werden müssen. Darüber hinaus ist das Verfahren sehr zeitaufwendig.

Nach dem in DE 3 626 210 beschriebenen Verfahren werden 1-Hydroxy-2-pyridone durch Umsetzung der entsprechenden 2-Pyrone mit Hydroxylamin oder einem Hydroxylammoniumsalz in Gegenwart von basischen Verbindungen wie Alkalicarbonat oder -hydrogencarbonat bei Temperaturen zwischen 50 °C und 120 °C hergestellt, wobei das Alkalicarbonat hierbei vorteilhaft in wenigstens äquimolarer Menge, bezogen auf das Hydroxylammoniumsalz, eingesetzt wird. Die Vorteile dieses Verfahrens gegenüber dem in DE 2 214 608 beschriebenen sind die erhöhte Wirtschaftlichkeit und die geringe Umweltbelastung. Nachteil des in DE 3 626 210 beschriebenen Verfahrens sind die im Vergleich mit DE 2 214 608 geringeren Ausbeuten, die zwischen 50 % und 60 % der Theorie, bezogen auf eingesetztes 2-Pyron, liegen.

Es wurde nun gefunden, daß die Ausbeute an 1-Hydroxy-2-pyridonen bei dem in DE 36 26 210 beschriebenen Verfahren durch Zusatz von organischen Säuren oder deren Salz gesteigert werden kann.

Die Erfindung betrifft ein Verfahren zur Herstellung von 1-Hydroxy-2-pyridonen der Formel I 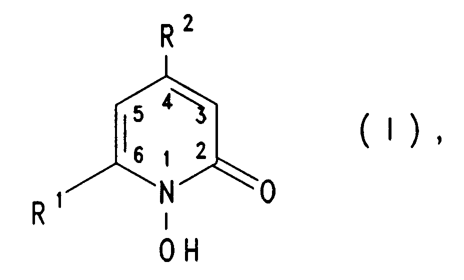 durch Umsetzung eines Pyrons der Formel II, 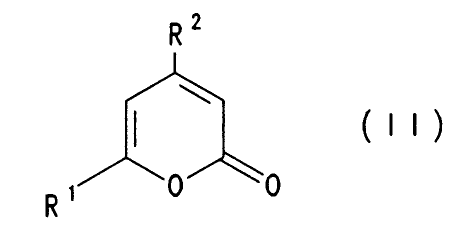 mit einem Hydroxylammoniumsalz in Gegenwart von basischen Verbindungen und Lösungsmitteln, das dadurch gekennzeichnet ist, daß man die Umsetzung in Gegenwart einer organischen Säure oder deren Salz durchführt und als basische Verbindungen Alkalicarbonat und/oder Alkalihydrogencarbonat in einer Menge von 0,8 bis 5 Äquivalenten, bezogen auf das Hydroxylammoniumsalz, einsetzt, dabei haben in den Formeln I und II die Reste R¹ und R² die nachstehenden Bedeutungen:
R¹ ist ein verzweigtes oder unverzweigtes Alkyl mit 1 bis 17 Kohlenstoffatomen, vorzugsweise 1 bis 12 Kohlenstoffatomen, insbesondere 1 bis 10 Kohlenstoffatomen,
   ein verzweigtes oder unverzweigtes Alkenyl mit 2 bis 17 Kohlenstoffatomen,
   ein Cycloalkyl mit 3 bis 8 Kohlenstoffatomen im Ring, vorzugsweise 6 Kohlenstoffatomen im Ring;
   wobei der genannte Cycloalkylrest unsubstituiert oder durch 1 bis 3 Alkylreste mit jeweils 1 bis 3 Kohlenstoffatomen substituiert ist,
   ein unsubstituierter oder im aromatischen Kern durch 1 bis 3 (C₁-C₆)Alkyl-, Benzyl-, (C₁-C₆)Alkoxy-, Phenoxy- oder Halogenreste substituierter Phenyl-, Phenyloxy-(C₁-C₄)alkyl- oder Phenyl-(C₁-C₄)alkylrest,
   wobei auch die als Substituent enthaltene Benzyl- oder Phenoxygruppe in gleicher Weise substituiert sein kann,
   wobei die genannten Cycloalkyl-, Phenyl-, Phenyloxy-(C₁-C₄)alkyl- oder Phenyl-(C₁-C₄)alkylreste direkt oder über eine Methylen- oder Ethylengruppe an den Pyridonring gebunden sind, und
R² ist Wasserstoffatom,
   ein Alkyl mit 1 bis 6 Kohlenstoffatomen,
   ein Alkenyl mit 2 bis 6 Kohlenstoffatomen oder
   ein Benzylrest,
   wobei die Alkyl-, Alkenyl- oder Benzylreste unsubstituiert oder in der bei R¹ angegebenen Weise substituiert sind.

Unter den Resten R¹ und R², die den Phenylrest enthalten, sind solche bevorzugt, in denen dieser Phenylkern nicht oder nur ein- oder zweimal substituiert ist. Bevorzugt unter den für R² genannten Resten sind Alkylreste mit 1 bis 4, insbesondere mit 1 bis 2 Kohlenstoffatomen, unter den Alkenylresten solche mit 2, 3 oder 4 Kohlenstoffatomen.

Ganz besonders bevorzugt wird eine Verbindung der Formel I hergestellt, darin bedeuten R¹ 2,4,4-Trimethylpentyl, Cyclohexyl, 4-(4-Chlorphenoxy)phenoxymethyl und R² Methyl.

Unter dem Begriff Halogenrest wird Fluor-, Chlor-, Brom- oder Jodatom verstanden. Alkyl steht für Reste, die sich beispielsweise von Methan, Ethan, Propan, Butan, Pentan, Hexan oder Heptan ableiten. Unter dem Begriff Alkenyl werden Reste verstanden, die sich beispielsweise von Ethen,
Propen, Buten, Penten, Hexen oder Hepten ableiten.

Beispiele für eine organische Säure oder deren Salz, die in dem erfindungsgemäßen Verfahren eingesetzt wird, sind Valeriansäure, Phenoxyessigsäure, Diphenylessigsäure, 4-n-Pentylbenzoesäure, 2-Methoxybenzoesäure, Essigsäure, Terephthalsäure, 4-Dimethylaminobenzoesäure, 3-Tolylsäure, 3,4-Dimethoxybenzoesäure, 3-Butoxybenzoesäure, 2-Chlorbenzoesäure, 4n-Heptylbenzoesäure, 4-Tolylsäure, 2,3-Dimethylbenzoesäure, Benzoesäure, Salicylsäure, Naphthoesäure, 2-Tolylsäure, 2,4,6-Trimethylbenzoesäure, 3-Methoxybenzoesäure, 4-t-Butylbenzoesäure, 2,5-Dimethylbenzoesäure, 4n-Butylbenzoesäure, Trifluoressigsäure oder Ionenaustauscher. Bevorzugte organische Säuren sind z.B. Benzoesäure, 4-tertiär-Butylbenzoesäure und/oder Trifluoressigsäure. Als Salze werden vorteilhaft Natrium- oder Kaliumsalz eingesetzt. Es werden 0,1 bis 40 Gewichtsprozent der organischen Säure, bezogen auf das Pyron der Formel II, eingesetzt, bevorzugt 5 bis 20 Gewichtsprozent, insbesondere 8 bis 12 Gewichtsprozent.

Das Hydroxylammoniumsalz wird in äquimolarer Menge, bezogen auf das Pyron der Formel II, eingesetzt; zur Beschleunigung der Reaktion kann es jedoch auch im Überschuß eingesetzt werden, wobei dann oft bessere Ausbeuten erhalten werden. Auch kann es zweckmäßig sein, das Hydroxylammoniumsalz in mehreren Portionen im Verlauf der Reaktion zuzusetzen. Als Hydroxylammoniumsalz können im wesentlichen alle Salze des Hydroxylamins, z.B. das Chlorid, das Sulfat oder das Acetat verwendet werden. Bevorzugt ist jedoch die Durch-führung der Umsetzung mit den leicht zugänglichen Hydroxylammoniumsulfat oder -chlorid.

Als basische Verbindungen werden bevorzugt Alkalicarbonat oder Alkalihydrogencarbonat in einer Menge von 0,8 bis 5 Äquivalenten, bezogen auf das Hydroxylammoniumsalz, im erfindungsgemäßen Verfahren eingesetzt, insbesondere 0,9 bis 1,1 Äquivalente.

Als Alkalicarbonat oder Alkalihydrogencarbonat kommen praktisch alle Carbonate und Bicarbonate der Alkalimetalle in Betracht, z.B. Li₂CO₃, Na₂CO₃, K₂CO₃, NaHCO₃, KHCO₃. Bevorzugt sind die Carbonate und Bicarbonate des Natriums und des Kaliums, besonders bevorzugt ist Na₂CO₃. Die Alkalicarbonate und Alkalibicarbonate können sowohl einzeln als auch in praktisch beliebiger Mischung zum Einsatz gelangen. Ihre Menge ist zweckmäßig der verwendeten Menge an Hydroxylammoniumsalz mindestens äquivalent, bei Verwendung eines Überschusses an Hydroxylammoniumsalz, kann jedoch auch eine geringere Menge Alkalicarbonat oder Alkalibicarbonat eingesetzt werden. Pro Mol Hydroxylammoniumchlorid ist beispielsweise 0,5 Mol Na₂CO₃ oder 1 Mol NaHCO₃ zu verwenden.

Zur Durchführung des erfindungsgemäßen Verfahrens mischt man vorteilhaft das Pyron der Formel II mit dem Hydroxylammoniumsalz und dem Alkalicarbonat und der organischen Säure und erwärmt den erhaltenen Kristallbrei, bis kein Pyron der Formel II mehr nachzuweisen ist; das entstandene Pyridon der Formel I wird nach Abtrennung der anorganischen Salze und der organischen Säure direkt, oder noch besser als Salz einer organischen Base, z.B. als Ethanolaminsalz, isoliert. Das erfindungsgemäße Verfahren wird bei einer Temperatur von 50 °C bis 120 °C, bevorzugt von 60 °C bis 105 °C, durchgeführt.

Die organische Säure wird durch Destillation oder Neutralisation mit anschließender Filtration oder Extraktion aus dem Reaktionsansatz entfernt. Die Aufarbeitung erfolgt beispielsweise bei Benzoesäure dadurch, daß der in Essigsäureethylester gelöste Ansatz mit Bicarbonatlösung oder mit Natronlauge gewaschen wird.
Beim Waschen mit Natronlauge muß darauf geachtet werden, daß die wäßrige Phase ausreichend verdünnt ist, und daß ein pH von ca. 8,0 nicht überschritten wird, da sonst Emulsionseffekte auftreten, die eine schlechte Phasentrennung bewirken. Die Basenbehandlung kann auch so erfolgen, daß man nach Beendigung der Reaktion zum Ansatz noch etwas festes NaCO₃ gibt und nachrührt. Benzoesäure und die sauren Verunreinigungen bilden dabei Natriumsalze. Der größte Teil der anfallenden Salze kann durch Absaugen und der Rest durch Waschen der organischen Phase mit Wasser entfernt werden.

Trifluoressigsäure kann auch durch Destillation unter verminderten Druck teilweise aus dem Reaktionsansatz entfernt werden.

Lösungsmittel werden in kleinen Mengen oder bis zu 50 Gewichtsprozent des gesamten Reaktionsansatzes zugesetzt. Bevorzugt beträgt die Menge 3 bis 15 Gewichtsprozent. Die Lösungsmittel können polar oder unpolar, mit Wasser mischbar oder nicht mischbar sein. Beispielweise können folgende Substanzen verwendet werden:
Wasser, niedermolekulare Alkohole wie Methanol, Ethanol oder Isopropanol, Ethylenglykol, Ethylenglykolmonomethylether, Propylenglykol, Säureamide wie Dimethylformamid, und Ester wie Ethylacetat, Ether wie Diisopropylether, chlorierte Kohlenwasserstoffe wie Chlorbenzol, Nitrile wie Acetonitril, Kohlenwasserstoffe aliphatischer, cycloaliphatischer oder aromatischer Natur wie Heptan oder Toluol.

Die erzielten Ausbeuten der Verbindung der Formel I betragen je nach verwendeter organischer Säure in der Regel von 60 % bis 77 %, bezogen auf das Pyron der Formel II.

Gegenüber dem Verfahren in DE 36 26 210 zeichnet sich das erfindungsgemäße Verfahren durch höhere Ausbeuten und durch höhere Wirtschaftlichkeit aus.

### Beispiele 1 bis 26

4-Methyl-6-(2,4,4-trimethylpentyl)-2-pyron, Hydroxylammoniumchlorid, Natriumcarbonat und jeweils 5 Gewichtsprozent der in Tabelle 1 genannten organischen Säuren werden in 200 ml Heptan und 2 ml Wasser suspendiert. Das Reaktionsgemisch wird 12 Stunden unter Rückflußbedingungen auf 95°C erwärmt. In der Tabelle 1 werden die Ausbeuten mit und ohne Zugabe einer organischen Säure genannt. Die Ausbeutebestimmung an 1-Hydroxy-4-methyl-6-(2,4,4-trimethylpentyl)-2-pyridon erfolgt durch Probennahme aus dem Reaktionsansatz und anschließende Hochdruckflüssigchromatographiebestimmung (HPLC). Die Ausbeute bezieht sich auf das eingesetzte Pyron.

**Tabelle 1**

| Beispiel-Nr. | Zusatz | Ausbeute [%] |
|---|---|---|
| 0 | ohne Zusatz | 58 |
| 1 | Valeriansäure | 61 |
| 2 | Phenoxyessigsäure | 61 |
| 3 | Diphenylessigsäure | 62 |
| 4 | 4-n-Pentylbenzoesäure | 63 |
| 5 | 2-Methoxybenzoesäure | 63 |
| 6 | Essigsäure | 64 |
| 7 | Terephthalsäure | 64 |
| 8 | 4-Dimethylamino-benzoesäure | 64 |
| 9 | 3-Tolylsäure | 64 |
| 10 | 3,4-Dimethoxybenzoesäure | 64 |
| 11 | 3-Butoxybenzoesäure | 65 |
| 12 | 2-Chlorbenzoesäure | 66 |
| 13 | 4n-Heptylbenzoesäure | 66 |
| 14 | 4-Tolylsäure | 67 |
| 15 | 2,3-Dimethylbenzoesäure | 67 |
| 16 | Benzoesäure | 68 |
| 17 | Salicylsäure | 68 |
| 18 | Naphthoesäure | 68 |
| 19 | 2-Tolylsäure | 68 |
| 20 | 2,4,6-Trimethylbenzoesäure | 68 |
| 21 | 3-Methoxybenzoesäure | 68 |
| 22 | Trifluoressigsäure | 68 |
| 23 | 2,5-Dimethylbenzoesäure | 70 |
| 24 | 4n-Butylbenzoesäure | 70 |
| 25 | 4-t-Butylbenzoesäure | 70 |
| 26 | Benzoesäure Natriumsalz | 66 |

Die HPLC-Bestimmung erfolgt unter folgenden Bedingungen:
- Säule:: Diol-Säule (Firma E. Merck, Darmstadt, FRG) 7 µm 250 mm/4mm
- Element:: Acetonitril mit 6 ml 85 %iger H₃PO₄ im Liter
- Flow:: 2 ml/min
- Detektor:: UV 198 mm (mit einer Bandbreite von 10 mm)
284 mm (mit einer Bandbreite von 4 mm)

### Beispiel 27

4-Methyl-6-(2,4,4-trimethylpentyl)-2-pyron wird wie in Beispiel 1 mit unterschiedlichen Mengen von Benzoesäure, jeweils bezogen auf die Ausgangsubstanzen, umgesetzt. Tabelle 2 zeigt die Ergebnisse.

**Tabelle 2**

| Gewichtsprozente Benzoesäure | Ausbeute [ %] |
|---|---|
| 3 | 68,4 |
| 5 | 68 |
| 7 | 69,4 |
| 9 | 70 |
| 13 | 71 |
| 20 | 72,3 |
| 30 | 71,8 |
| 40 | 70,8 |

Die Bestimmung erfolgt durch HPLC-Messung aus der Reaktionslösung.

### Beispiel 28

Die Umsetzung erfolgt wie in Beispiel 1 mit unterschiedlichen Mengen von 4-t-Butylbenzoesäure, jeweils bezogen auf die Ausgangssubstanz. Tabelle 3 zeigt die Ergebnisse.

| Gewichtsprozente 4-t-Butylbenzoesäure | Ausbeute [%] |
|---|---|
| 5 | 70 |
| 10 | 73,1 |
| 20 | 76,9 |
| 30 | 71,8 |

Die Bestimmung erfolgt durch HPLC-Messung aus der Reaktionslösung.

### Beispiel 29

222,3 g (1 Mol) 4-Methyl-6-(2,4,4-trimethylpentyl)-2-pyron, 195,8 g (2,84 Mol) Hydroxylammoniumchlorid, 149,2 g (1,41 Mol) Na₂CO₃, 21,9 g (0,18 Mol) Benzoesäure und 2 ml H₂O und 200 ml Heptan werden vereinigt und 12 Stunden unter Rückfluß gekocht. Dann gibt man 21,9 g Na₂CO₃ (0,207 Mol) in Portionen vorsichtig zu und kocht eine weitere Stunde unter Rückfluß. Noch warm filtriert man ab und wäscht die Salze mit Essigsäureethyl-ester (EtOAc). Die organische Phase wird eingedampft und der Rückstand in 1000 ml EtOAc aufgenommen, dreimal mit je 1000 ml Wasser gewaschen und die wäßrigen Phasen einmal mit 300 ml EtOAc extrahiert. Nach dem Trocknen der vereinigten organischen Phasen über Na₂SO₄ wird auf etwa 600 ml eingedampft und durch Zugabe von 49 ml Ethanolamin das 1-Hydroxy-4-methyl-6-(2,4,4-trimethylpentyl)-2-pyridon ausgefällt. Vor dem Absaugen kühlt man die Fällung über Nacht im Kühlschrank auf 5-10°C ab.
Ausbeute: 175 - 178 g (65,2 bis 66,3 %)
Schmelzpunkt: 124°C

### Beispiel 30

204,2 g (0,9 Mol) 4-Methyl-6-(2,4,4-trimethylpentyl)-2-pyron (98 %), 179,4 g (2,56 Mol) Hydroxylammoniumchlorid (99 %), 135,9 g (1,27 Mol) Natriumcarbonat (99 %) werden in 200 ml n-Heptan und 2 ml Wasser suspendiert und mit 12,6 ml (0,16 Mol) Trifluoressigsäure versetzt. Dabei tritt starke CO₂-Entwicklung ein. Unter Rühren wird das Reaktionsgemisch 15 Stunden auf 95°C erwärmt. Man läßt abkühlen und extrahiert mit 300 ml 0,1 N NaOH. Heptan und verbleibende Trifluoressigsäure werden unter vermindertem Druck abdestilliert, der Rückstand wird in 400 ml Essigsäureethylester gelöst, und bei etwa 50°C wird die erhaltene Lösung mit 48,6 g (0,79 Mol) Ethanolamin versetzt. Nach dem Animpfen läßt man die Lösung abkühlen. Die entstandenen Kristalle werden abgesaugt, mit wenig kaltem Ethylacetat gewaschen und getrocknet. Die Ausbeute an 1-Hydroxy-4-methyl-6-(2,4,4-trimethylpentyl)-2-pyridon (Ethanolaminsalz) beträgt 169,3 g (63 %).

## Patentansprüche

1. Verfahren zur Herstellung von 1-Hydroxy-2-pyridonen der Formel I 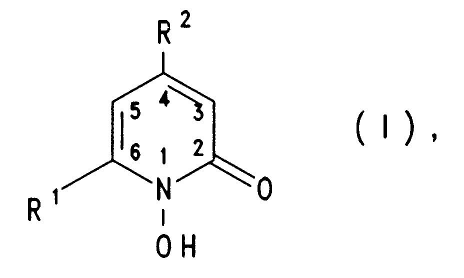 durch Umsetzung eines Pyrons der Formel II, 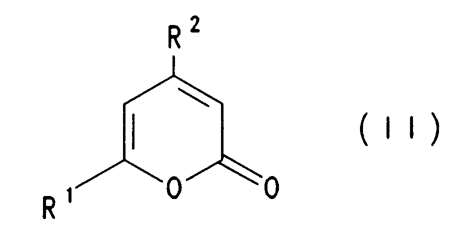 mit einem Hydroxylammoniumsalz in Gegenwart von basischen Verbindungen und Lösungsmitteln,
dadurch gekennzeichnet, daß man die Umsetzung in Gegenwart einer organischen Säure oder deren Salz durchführt und als basische Verbindungen Alkalicarbonat und/oder Alkalihydrogencarbonat in einer Menge von 0,8 bis 5 Äquivalenten, bezogen auf das Hydroxylammoniumsalz, einsetzt.
dabei haben in den Formeln I und II die Reste R¹ und R² die nachstehenden Bedeutungen:
R¹ ist ein verzweigtes oder unverzweigtes Alkyl mit 1 bis 17 Kohlenstoffatomen,
ein verzweigtes oder unverzweigtes Alkenyl mit 2 bis 17 Kohlenstoffatomen,
ein Cycloalkyl mit 3 bis 8 Kohlenstoffatomen im Ring,
wobei der genannte Cycloalkylrest unsubstituiert oder durch 1 bis 3 Alkylreste mit jeweils 1 bis 3 Kohlenstoffatomen substituiert ist,
ein unsubstituierter oder im aromatischen Kern durch 1 bis 3 (C₁-C₆)Alkyl-, Benzyl-, (C₁-C₆)Alkoxy-, Phenoxy- oder Halogenreste substituierter Phenyl-, Phenyloxy-(C₁-C₄)alkyl- oder Phenyl-(C₁-C₄)alkylrest,
wobei auch die als Substituent enthaltene Benzyl- oder Phenoxygruppe in gleicher Weise substituiert sein kann,
wobei die genannten Cycloalkyl-, Phenyl-, Phenyloxy-(C₁-C₄)alkyl- oder Phenyl-(C₁-C₄)alkylreste direkt oder über eine Methylen- oder Ethylengruppe an den Pyridonring gebunden sind, und
R² ist ein Wasserstoffatom,
ein Alkyl mit 1 bis 6 Kohlenstoffatomen,
ein Alkenyl mit 2 bis 6 Kohlenstoffatomen oder
ein Benzylrest,
wobei die Alkyl-, Alkenyl- oder Benzylreste unsubstituiert oder in der bei R¹ angegebenen Weise substituiert sind.

2. Verfahren nach Anspruch 1, dadurch gekennzeichnet, daß eine Verbindung der Formel I hergestellt wird, wobei R¹ für Alkyl mit 1 bis 10 Kohlenstoffatomen, Cyclohexyl oder Phenoxymethyl steht und R² für (C₁-C₄)Alkyl steht, oder die Reste R¹ oder R², die den Phenylkern enthalten, unsubstituiert sind oder höchstens 2 Substituenten tragen.

3. Verfahren nach Anspruch 1, dadurch gekennzeichnet, daß eine Verbindung der Formel I hergestellt wird, wobei R¹ für 2,4,4-Trimethylpentyl, Cyclohexyl oder 4-(4-Chlorphenoxy)-phenoxymethyl steht und R² für Methyl steht.

4. Verfahren nach einem oder mehreren der Ansprüche 1 bis 3, dadurch gekennzeichnet, daß man als organische Säure Benzoesäure oder Trifluoressigsäure in einer Menge von 0,1 bis 40 Gewichtsprozenten, insbesondere von 5 bis 20 Gewichtsprozenten, bezogen auf das Pyron der Formel II, und als basische Verbindungen Alkalicarbonat und/oder Alkalihydrogencarbonat in einer Menge von 0,9 bis 1,1 Äquivalente bezogen auf das Hydroxylammoniumsalz einsetzt.

5. Verfahren nach einem oder mehreren der Ansprüche 1 bis 4, dadurch gekennzeichnet, daß als basische Verbindung Natriumcarbonat, Kaliumcarbonat, Natriumhydrogencarbonat oder Kaliumhydrogencarbonat, insbesondere Natriumcarbonat und als Hydroxylammoniumsalz Hydroxylammoniumchlorid eingesetzt wird.

6. Verfahren nach einem oder mehreren der Ansprüche 1 bis 5, dadurch gekennzeichnet, daß bei einer Temperatur von 50 °C bis 120 °C, vorzugsweise 60°C bis 105°C, gearbeitet wird.

7. Verfahren nach einem oder mehreren der Ansprüche 1 bis 6, dadurch gekennzeichnet, daß ein Lösungsmittel, in einer Menge von bis zu 50 Gewichtsprozent des gesamten Reaktionsansatzes, insbesondere in einer Menge von 3 bis 15 Gewichtsprozent, eingesetzt wird.

8. Verfahren nach Anspruch 7, dadurch gekennzeichnet, daß als Lösungsmittel Heptan oder Toluol eingesetzt wird.

9. Verfahren nach einem oder mehreren der Ansprüche 1 bis 8, dadurch gekennzeichnet, daß der Reaktionsansatz anschließend mit Natriumcarbonat oder Natronlauge gewaschen wird.

10. Verfahren nach einem oder mehreren der Ansprüche 1 bis 8, dadurch gekennzeichnet, daß das Lösungsmittel oder die organische Säure oder Lösungsmittel und organische Säure anschließend durch Destillation entfernt wird.

## Claims

1. A process for the preparation of a 1-hydroxy-2-pyridone of the formula I 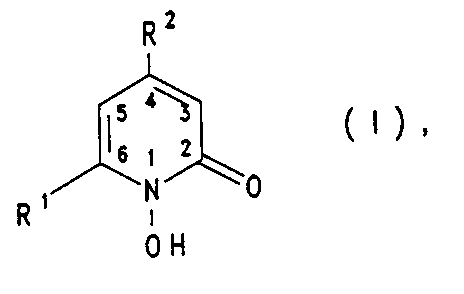 by reaction of a pyrone of the formula II 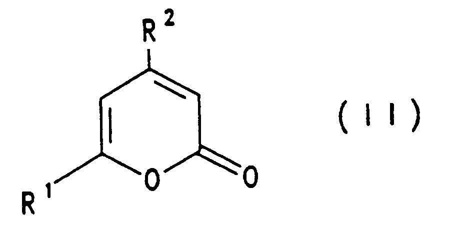 with a hydroxylammonium salt in the presence of basic compounds and solvents,
which comprises carrying out the reaction in the presence of an organic acid or a salt thereof and employing as the basic compounds an alkali metal carbonate and/or alkali metal bicarbonate in an amount of 0.8 to 5 equivalents with respect to the hydroxylammonium salt,
the radicals R¹ and R² in the formulae I and II having the following meanings:
R¹ is a branched or unbranched alkyl having 1 to 17 carbon atoms,
a branched or unbranched alkenyl having 2 to 17 carbon atoms,
a cycloalkyl having 3 to 8 carbon atoms in the ring, the cycloalkyl radical mentioned being unsubstituted or substituted by 1 to 3 alkyl radicals each having 1 to 3 carbon atoms,
a phenyl, phenyloxy(C₁-C₄)alkyl or phenyl-(C₁-C₄) alkyl radical which is unsubstituted or substituted in the aromatic nucleus by 1 to 3 (C₁-C₆)-alkyl, benzyl, (C₁-C₆)alkoxy, phenoxy or halogen radicals,
it also being possible for the benzyl or phenoxy group present as a substituent to be substituted in the same manner,
the cycloalkyl, phenyl, phenyloxy-(C₁-C₄)alkyl or phenyl-(C₁-C₄)alkyl radicals mentioned being bonded to the pyridone ring directly or via a methylene or ethylene group, and
R² is a hydrogen atom,
an alkyl having 1 to 6 carbon atoms,
an alkenyl having 2 to 6 carbon atoms or
a benzyl radical,
the alkyl, alkenyl or benzyl radicals being unsubstituted or substituted in the manner described for R¹.

2. The process as claimed in claim 1, wherein a compound of the formula I is prepared in which R¹ is alkyl having 1 to 10 carbon atoms, cyclohexyl or phenoxymethyl and R² is (C₁-C₄)alkyl, or the radicals R¹ or R², which contain the phenyl nucleus, are unsubstituted or carry not more than 2 substituents.

3. The process as claimed in claim 1, wherein a compound of the formula I is prepared in which R¹ is 2,4,4-trimethylpentyl, cyclohexyl or 4-(4-chlorophenoxy)-phenoxymethyl and R² is methyl.

4. The process as claimed in one or more of claims 1 to 3, wherein benzoic acid or trifluoroacetic acid is employed as the organic acid in an amount of 0.1 to 40 percent by weight, in particular of 5 to 20 percent by weight, with respect to the pyrone of the formula II, and an alkali metal carbonate and/or alkali metal bicarbonate are employed as the basic compounds in an amount of 0.9 to 1.1 equivalents with respect to the hydroxylammonium salt.

5. The process as claimed in one or more of claims 1 to 4, wherein sodium carbonate, potassium carbonate, sodium bicarbonate or potassium bicarbonate, in particular sodium carbonate, is employed as the basic compound and hydroxylammonium chloride is employed as the hydroxylammonium salt.

6. The process as claimed in one or more of claims 1 to 5, which is carried out at a temperature of 50°C to 120°C, preferably 60°C to 105°C.

7. The process as claimed in one or more of claims 1 to 6, wherein a solvent is employed in an amount of up to 50 percent by weight of the total reaction batch, in particular in an amount of 3 to 15 percent by weight.

8. The process as claimed in claim 7, wherein heptane or toluene is employed as the solvent.

9. The process as claimed in one or more of claims 1 to 8, wherein the reaction batch is subsequently washed with sodium carbonate or sodium hydroxide solution.

10. The process as claimed in one or more of claims 1 to 8, wherein the solvent or the organic acid or solvent and organic acid is or are subsequently removed by distillation.

## Revendications

1. Procédé pour la préparation des 1-hydroxy-2-pyridones de formule I 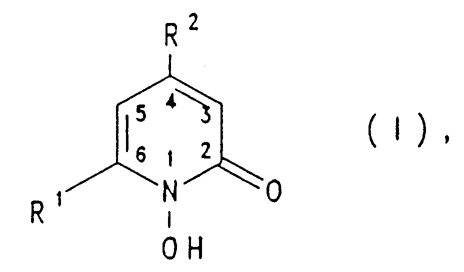 par la réaction d'une pyrone de formule II 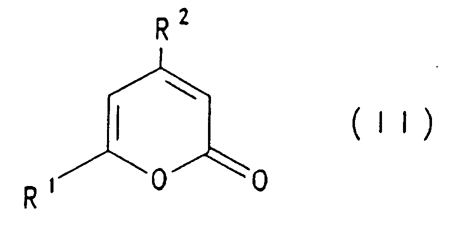 avec un sel d'hydroxylammonium, en présence de composés basiques et de solvants, caractérisé en ce que l'on effectue la réaction en présence d'un acide organique ou d'un sel de celui-ci, et on utilise, en tant que composés basiques, un carbonate de métal alcalin et/ou un hydrogénocarbonate de métal alcalin, en une quantité de 0,8 à 5 équivalents, par rapport au sel d'hydroxylammonium,
dans les formules I et II, les radicaux R¹ et R² ayant les significations suivantes:
R¹ est un groupe alkyle ramifié ou non ramifié ayant de 1 à 17 atomes de carbone,
un groupe alcényle ramifié ou non ramifié ayant de 2 à 17 atomes de carbone,
un groupe cycloalkyle ayant de 3 à 8 atomes de carbone dans le cycle,
ledit radical cycloalkyle étant non substitué ou substitué par 1 à 3 groupes alkyle ayant chacun de 1 à 3 atomes de carbone,
un groupe phényle, phényloxy-alkyle(C₁-C₄) ou phényl-alkyle(C₁-C₄) non substitué ou substitué sur le noyau aromatique par 1 à 3 atomes d'halogène ou radicaux alkyle en C₁-C₆, benzyle, alcoxy en C₁-C₆ ou phénoxy, le groupe benzyle ou phénoxy contenu en tant que substituant pouvant également être substitué de la même façon,
lesdits radicaux cycloalkyle, phényle, phényloxy-alkyle(C₁-C₄) ou phénylalkyle(C₁-C₄) étant reliés directement ou par un groupe méthylène ou éthylène au cycle pyridone, et
R² est un atome d'hydrogène,
un groupe alkyle ayant de 1 à 6 atomes de carbone,
un groupe alcényle ayant de 2 à 6 atomes de carbone ou le groupe benzyle,
les groupes alkyle, alcényle et benzyle étant non substitués ou substitués de la façon indiquée pour R¹.

2. Procédé selon la revendication 1, caractérisé en ce que l'on prépare un composé de formule I dans lequel R¹ représente un groupe alkyle ayant de 1 à 10 atomes de carbone, cyclohexyle ou phénoxyméthyle, et R² représente un groupe alkyle en C₁-C₄, ou les radicaux R¹ ou R² qui contiennent les noyaux phényle sont non substitués ou portent 2 substituants au maximum.

3. Procédé selon la revendication 1, caractérisé en ce que l'on prépare un composé de formule I dans lequel R¹ représente le groupe 2,4,4-triméthylpentyle, cyclohexyle ou 4-(4-chlorophénoxy)phénoxyméthyle, et R² représente le groupe méthyle.

4. Procédé selon une ou plusieurs des revendications 1 à 3, caractérisé en ce que l'on utilise comme acide organique l'acide benzoïque ou l'acide trifluoroacétique, en une proportion de 0,1 à 40 % en poids, en particulier de 5 à 20 % en poids, par rapport à la pyrone de formule II, et, en tant que composés basiques, un carbonate de métal alcalin et/ou un hydrogénocarbonate de métal alcalin, en une quantité de 0,9 à 1,1 équivalent, par rapport au sel d'hydroxylammonium.

5. Procédé selon une ou plusieurs des revendications 1 à 4, caractérisé en ce que l'on utilise en tant que composé basique le carbonate de sodium, le carbonate de potassium, l'hydrogénocarbonate de sodium ou l'hydrogénocarbonate de potassium, en particulier le carbonate de sodium, et, en tant que sel d'hydroxylammonium, le chlorure d'hydroxylammonium.

6. Procédé selon une ou plusieurs des revendications 1 à 5, caractérisé en ce que l'on opère à une température de 50 à 120°C, de préférence de 60 à 105°C.

7. Procédé selon une ou plusieurs des revendications 1 à 6, caractérisé en ce que l'on utilise un solvant en une proportion allant jusqu'à 50 % en poids du mélange réactionnel total, en particulier en une proportion de 3 à 15 % en poids.

8. Procédé selon la revendication 7, caractérisé en ce que l'on utilise comme solvant l'heptane ou le toluène.

9. Procédé selon une ou plusieurs des revendications 1 à 8, caractérisé en ce que le mélange réactionnel est ensuite lavé avec du carbonate de sodium ou une solution d'hydroxyde de sodium.

10. Procédé selon une ou plusieurs des revendications 1 à 8, caractérisé en ce que le solvant ou l'acide organique ou la solvant et l'acide organique sont ensuite éliminés par distillation.
